(19)

Europäisches Patentamt
European Patent Office
Office européen des brevets

(11) **EP 4 223 413 A1**

(12) **EUROPEAN PATENT APPLICATION**
published in accordance with Art. 153(4) EPC

(43) Date of publication:
**09.08.2023 Bulletin 2023/32**

(21) Application number: **21875679.9**

(22) Date of filing: **29.09.2021**

(51) International Patent Classification (IPC):
**B01J 29/44** (2006.01)          **A61L 9/00** (2006.01)
**A61L 9/01** (2006.01)

(52) Cooperative Patent Classification (CPC):
**A61L 9/00; A61L 9/01; B01J 29/44**

(86) International application number:
**PCT/JP2021/035817**

(87) International publication number:
**WO 2022/071379 (07.04.2022 Gazette 2022/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.10.2020 JP 2020167711**

(71) Applicant: **Mitsui Chemicals, Inc.**
**Tokyo 104-0028 (JP)**

(72) Inventors:
• **KAWAHARA Jun**
  **Sodegaura-shi, Chiba 299-0265 (JP)**
• **NAGAI Naoshi**
  **Sodegaura-shi, Chiba 299-0265 (JP)**
• **OKABE Akihiro**
  **Tokyo 105-7122 (JP)**
• **SAITO Susumu**
  **Sodegaura-shi, Chiba 299-0265 (JP)**
• **TAHARA, Shuji**
  **Sodegaura-shi, Chiba 299-0265 (JP)**

(74) Representative: **Hoffmann Eitle**
**Patent- und Rechtsanwälte PartmbB**
**Arabellastraße 30**
**81925 München (DE)**

(54) **ODOR REMOVAL CATALYST AND USE THEREFOR**

(57)    An object of the present invention is to provide a deodorizing catalyst capable of rapidly adsorbing and removing an odor in a space such as the inside of an automobile, continuously reducing the odor for a long period of time by decomposing the adsorbed odor component, and preventing the odor from returning, and to provide a deodorant composition and a deodorizing product using the deodorizing catalyst.

The deodorizing catalyst of the present invention is a deodorizing catalyst containing a composite oxide and a metal component containing Pt supported on the composite oxide, and having a degree of reduction represented by the following formula (1) in a range of 60% to 86%:

$$\text{degree of reduction } (\%) = 100 - [X/Y] \times 100 \quad (1)$$

(1)

(in the formula (1), X represents a peak area obtained from a thermal conductivity detector (TCD) when the catalyst is dried and reduced by heating from 40°C to 400°C, and Y represents a peak area obtained from the TCD when an oxidation catalyst obtained by heat-treating the catalyst in an air or under oxygen atmosphere at 150°C for 2 hours and then cooling the catalyst to 40°C or lower is reduced by heating from 40°C to 400°C).

**(Cont. next page)**

[Fig. 1]

CHANGE IN PROPIONALDEHYDE CONCENTRATION OF EXAMPLE 4

**Description**

TECHNICAL FIELD

The present invention relates to a deodorizing catalyst and uses thereof. Specifically, the present invention relates to a deodorizing catalyst comprising a metal component containing Pt supported on a composite oxide support, and uses thereof.

BACKGROUND ART

Various odors generated in the environment (for example, exhaust gas, odors of garbage and foods, odors discharged from newly constructed houses and buildings) are large social issues, and an effective method for coping with each environment is required. In particular, odors in enclosed spaces such as residences, public facilities such as hotels and restaurants, and transportation such as trains and automobiles are a problem, but it is difficult to install special devices (for example, ozone deodorization, hypochlorite deodorization) that pose safety concerns because people coexist there, and easy-to-use adsorbents are suitably used. However, when the adsorbent is continuously used, it breaks beyond its adsorption capacity, so that it is necessary to frequently replace the adsorbent. Further, particularly on a day when the temperature is high, there is a problem that the odor component once adsorbed is desorbed to generate a malodor.

As a technique for adsorbing and decomposing odor components in the air, a photocatalyst, a Pt-supported silica catalyst and so on are known to be effective in decomposing volatile organic compounds (VOC) in the air.

Patent Literature 1 teaches that a catalyst comprising a composite of platinum and ruthenium supported on porous silica is capable of oxidatively decomposing ethylene or a mercaptan compound.

Patent Literature 2 discloses ZSM-5 doped with palladium as an adsorbent for a volatile organic compound (VOC) derived from an organic material, and indicates that the ZSM-5 can adsorb ethylene.

Patent Literature 3 describes a deodorant in which a chemically impregnated layer of, for example, aniline is provided on a surface of a porous support and a catalyst component such as ruthenium, platinum, or palladium is supported thereon, and Patent Literature 4 describes a deodorant in which a metal oxidation catalyst is supported on activated carbon.

CITATION LIST

PATENT LITERATURE

Patent Literature 1: WO2019/027057
Patent Literature 2: JP2015-213908A
Patent Literature 3: JP2002-200150A
Patent Literature 4: JP2000-312710A

SUMMARY OF INVENTION

TECHNICAL PROBLEM

The photocatalysts and Pt-supported silica catalysts described above are mainly intended for low-boiling-point substances having a small molecular weight (for example, formaldehyde), and according to the study by the present inventors, it cannot be said that the technique is necessarily sufficient for high-boiling-point components having a high odor intensity (for example, nonenal).

The catalyst disclosed in Patent Literature 1 is insufficient in the ability to decompose typical odorants such as ethyl acetate.

The adsorbent disclosed in Patent Literature 2 can be continuously used for adsorption and removal of VOC for several days, and after use, can be regenerated by heating in the air and reused, but cannot be used for a long period of time without being regenerated by high-temperature heating.

Both of the deodorants disclosed in Patent Literature 3 and Patent Literature 4 are insufficient in deodorizing effect, and the deodorant using activated carbon was excellent in initial adsorption but sufficient deodorizing effect was not obtained over time.

Under such circumstances, the present inventors have pursued a deodorizing means which is highly safe and can be stably used for a long period of time, and more specifically, have pursued an adsorbent which decomposes an adsorbed odor component and can be continuously used.

That is, an object of the present invention is to provide a deodorizing catalyst capable of rapidly adsorbing and removing an odor in a space such as the inside of an automobile, continuously reducing the odor for a long period of time by decomposing the adsorbed odor component, and preventing the odor from returning, and to provide a deodorant composition and a deodorizing product using the deodorizing catalyst.

SOLUTION TO PROBLEM

As a result of intensive studies in view of the above circumstances, the present inventors have found that a catalyst in which a metal containing Pt is supported on a composite oxide and which has a specific degree of reduction can maintain an excellent odor removal effect for a long period of time, thereby completing the present invention.

The present invention relates to, for example, the following items [1] to [12]:

[1] A deodorizing catalyst comprising a composite oxide, and a metal component containing Pt supported on the composite oxide, wherein the catalyst has a degree of reduction represented by the following formula (1) in a range of 60% to 86% :

$$\text{degree of reduction (\%)} = 100 - [X/Y] \times 100 \quad (1)$$

(in the formula (1), X represents a peak area obtained from a thermal conductivity detector (TCD) when the catalyst is dried and reduced by heating from 40°C to 400°C, and Y represents a peak area obtained from the TCD when an oxidation catalyst obtained by heat-treating the catalyst in an air or under oxygen atmosphere at 150°C for 2 hours and then cooling the catalyst to 40°C or lower is reduced by heating from 40°C to 400°C).

[2] The deodorizing catalyst according to [1], wherein the metal component comprises Pt and at least one metal element selected from Re, Fe, Ru, Co, Rh, Ir, Ni, Pd, Cu, Ag, and Au.

[3] The deodorizing catalyst according to [1] or [2], wherein the metal component comprises an alloy containing Pt and at least one metal element selected from Re, Fe, Ru, Co, Rh, Ir, Ni, Pd, Cu, Ag, and Au.

[4] The deodorizing catalyst according to [3], wherein the alloy forms a solid solution.

[5] The deodorizing catalyst according to any one of [1] to [4], wherein the composite oxide is MFI type zeolite.

[6] The deodorizing catalyst according to [5], wherein the MFI type zeolite has a silica/alumina ratio ($SiO_2/Al_2O_3$ (mol/mol)) of 40 or less.

[7] A deodorant composition comprising the deodorizing catalyst according to any one of [1] to [6] and a chemical adsorbent.

[8] A deodorizing product comprising the deodorizing catalyst according to any one of [1] to [6] or the deodorant composition according to [7].

[9] The deodorizing product according to [8], wherein the deodorizing product is a deodorizing fiber, a deodorizing coating material, or a deodorizing sheet.

[10] A upholstery material, a curtain, a carpet, a tile, a wallpaper, or an interior material for a vehicle, using the deodorizing product according to [8] or [9].

[11] An antibacterial/antiviral deodorizing product comprising the deodorizing catalyst according to any one of [1] to [6] or the deodorant composition according to [7].

[12] A upholstery material, a curtain, a carpet, a tile, a wallpaper, or an interior material for a vehicle, using the antibacterial/antiviral deodorizing product according to [11].

ADVANTAGEOUS EFFECTS OF INVENTION

The deodorizing catalyst of the present invention has high safety, adsorbs odor components, and decomposes the adsorbed odor components, thereby continuously reducing the odor for a long period of time without causing a problem of releasing the odor components once adsorbed with, for example, a temperature rise. The deodorizing catalyst of the present invention can continuously reduce an odor for a long period of time by being installed in a space having an odor, and can effectively deodorize, for example, the inside of a vehicle such as an automobile, and a room.

The deodorizing catalyst of the present invention can be used as a deodorant as it is, or can be used in various forms such as a composition, a resin-coated material, an interior sheet material, and an automobile inner panel containing the same as a component that provides an odor removal effect. According to the present invention, odorants such as aldehydes, carboxylic acids, and esters can be suitably decomposed and removed by using the deodorizing catalyst according to the present invention and a product containing the same. Further, according to the present invention, it is possible to provide a deodorizing method capable of decomposing and removing odorants safely and sustainably for a long period of time.

Further, the deodorizing catalyst of the present invention has excellent antibacterial and antiviral properties in addition to excellent deodorizing effects. Therefore, the deodorizing catalyst of the present invention and the product containing the same can be suitably used in uses where odor decomposition/removal and/or antibacterial/antiviral properties are required.

BRIEF DESCRIPTION OF DRAWINGS

[Figure 1] Figure 1 is a graph which shows changes over time in propionaldehyde concentration in a continuous decomposition test of Example 4.
[Figure 2] Figure 2 is a graph which shows changes over time in carbon dioxide concentration during the continuous decomposition test of Example 4.
[Figure 3] Figure 3 is a graph which shows changes over time in ethyl acetate concentration in a continuous decomposition test of Example 5.
[Figure 4] Figure 4 is a graph which shows changes over time in carbon dioxide concentration during the continuous decomposition test of Example 5.
[Figure 5] Figure 5 is a graph which shows changes over time in trimethylamine concentration in a continuous decomposition test of Example 6.
[Figure 6] Figure 6 is a graph which shows changes over time in carbon dioxide concentration during the continuous decomposition test of Example 6.
[Figure 7] Figure 7 is a graph which shows changes over time in methyl mercaptan concentration in a continuous decomposition test of Example 7.
[Figure 8] Figure 8 is a graph which shows changes over time in carbon dioxide concentration during the continuous decomposition test of Example 7.
[Figure 9] Figure 9 is a graph which shows changes over time in toluene concentration in a continuous decomposition test of Example 8.
[Figure 10] Figure 10 is a graph which shows changes over time in carbon dioxide concentration during the continuous decomposition test of Example 8.
[Figure 11] Figure 11 is a graph which shows changes over time in each odorant concentration in a mixed odorant decomposition test of Example 9.
[Figure 12] Figure 12 is a graph which shows the average decomposition rate of each odorant after 30 hours in a mixed odorant decomposition test of Examples 9 and 10 and Comparative Examples 3 and 4.
[Figure 13] Figure 13 is a graph which shows the relationship between odorant concentration and odor intensity.

DESCRIPTION OF EMBODIMENTS

The present invention will be specifically described hereinbelow.

<Deodorizing catalyst>

The deodorizing catalyst of the present invention is a deodorizing catalyst comprising a composite oxide and a metal component containing Pt supported on the composite oxide, and has a degree of reduction within a specific range of 60% to 80%.

Composite oxide

The composite oxide used in the present invention is capable of supporting a metal component, and is preferably a porous composite oxide.
The composite oxide is not particularly limited as long as it can support a metal component, and examples thereof include calcium titanate, aluminum silicate, and magnesium aluminate. Among these, zeolite (crystalline aluminosilicate) is preferred, and MFI type zeolite is particularly preferred.
When the composite oxide is MFI type zeolite, the silica/alumina ratio ($SiO_2/Al_2O_3$ (mol/mol)) is usually 45 or less, preferably 40 or less, and more preferably in the range of 20 to 30. It is preferable that the silica/alumina ratio is in such a range because the adsorption effect of, for example, odorants is excellent. The average particle size is more preferably in the range of 10 nm to 20 $\mu$m, particularly preferably in the range of 10 nm to 10 $\mu$m.

Metal component

The deodorizing catalyst of the present invention supports one or more metal components containing Pt (platinum). Examples of metals other than Pt contained in the metal component include, but are not limited to, Re, Fe, Ru, Co, Rh, Ir, Ni, Pd, Cu, Ag, and Au. The supported metal species may be Pt alone or may be a combination of Pt and another metal, and preferably Pt alone or a combination of Pt and at least one selected from Re, Fe, Ru, Co, Rh, Ir, Ni, Pd, Cu, Ag, and Au. The proportion of Pt in the entire supported metals (total of Pt and other metals) is usually 1 mol % or more, preferably 10 mol % or more.

The deodorizing catalyst of the present invention has a degree of reduction determined by the following formula (1) in the range of 60% to 86%, preferably 70% to 86%, more preferably 80% to 86%. The degree of reduction means the proportion of metal components in a reduced state in all metal components.

$$\text{degree of reduction (\%)} = 100 - [X/Y] \times 100 \qquad (1)$$

(In the formula (1), X represents a peak area obtained from a thermal conductivity detector (TCD) when the catalyst is reduced by heating from 40°C to 400°C, and Y represents a peak area obtained from the TCD when an oxidation catalyst obtained by heat-treating the catalyst in an air or under oxygen atmosphere at 150°C for 2 hours and then cooling the catalyst to 40°C or lower is reduced by heating from 40°C to 400°C.)

That is, in the deodorizing catalyst of the present invention, both a metal component in a reduced state (oxidation number is 0) and a reducible metal component such as a metal salt compound (oxidation number of the metal is 1 or more) are present, and the metal component in a reduced state is in the range of 60 to 86% of all supported metal components.

By satisfying the degree of reduction in such a range, the deodorizing catalyst of the present invention exhibits higher odor removal performance than a catalyst having an excessively low degree of reduction or a catalyst having an excessively high degree of reduction even when the amounts of all metal components are the same.

The metal component in a reduced state is supported on the composite oxide in the form of a simple metal or an alloy. In a case where the metal component in a reduced state contains Pt and another metal, Pt and the other metal may each be present as a simple metal or as an alloy, but at least a part of Pt and the other metal preferably form an alloy, and the alloy more preferably forms a solid solution. In the case of an alloy state in which Pt and the other metal form a solid solution, the removal performance per amount of Pt used is increased, which is preferable.

The amount of the metal component in the deodorizing catalyst is usually in the range of 0.01 to 10% by mass, preferably 0.01 to 5% by mass, and more preferably 0.01 to 1% by mass in terms of metal of the entire metal component. When the loading of the metal component is in such a range, a sufficient odor removal effect can be achieved, which is preferable.

The deodorizing catalyst of the present invention has an excellent deodorizing effect, and can particularly suitably reduce and remove odorants such as aldehydes, carboxylic acids, and esters. Specifically, the deodorizing catalyst of the present invention quickly reduces odorants in a surrounding gas by adsorbing the odorants in the pores of a composite oxide, and decomposes at least a part of the adsorbed odorants by the metal component containing Pt supported on the composite oxide. Therefore, the deodorizing catalyst of the present invention is less likely to cause the problem that odorants are released and odors are diffused even when exposed to a high temperature after adsorbing the odorants and the adsorbed odorants are decomposed and diffused, whereby the adsorption ability is not remarkably reduced, and the catalyst can be used for deodorization over a long period of time. Further, since the deodorizing catalyst of the present invention has antibacterial/antiviral properties, and is particularly excellent in antibacterial properties, it can be suitably used in uses where antibacterial/antiviral properties are required.

<Method for producing deodorizing catalyst>

As a method for producing the deodorizing catalyst of the present invention, a method in which one or more metal components containing Pt (platinum) are supported on the above-mentioned composite oxide so that the degree of reduction is in the range of 60% to 86% can be adopted without any particular limitations.

Examples of the method for producing a deodorizing catalyst include a method in which a metal containing Pt, a metal compound, or a mixture thereof is supported on a composite oxide by, for example, impregnation, physical mixing, an ion exchange, or a pore-filling, and the composite oxide is reduced as necessary.

Preferred is a method in which a composite oxide such as zeolite is impregnated with a solution or dispersion of Pt and, if necessary a compound of another metal, dried if necessary, and then reduced.

Examples of the Pt compound used for impregnation include platinum chloride, platinum oxide, platinum nitrate, platinum diamino dinitrite, platinum acetate, and platinum oxalate. Examples of the compound of another metal include metal salts and metal oxides. Furthermore, the metal compound used for impregnation may be a double salt containing Pt and other metals, for example.

The reduction of the metal compound supported by impregnation can be carried out, for example, by a reduction method by thermal decomposition, a reduction method using a gaseous reducing agent such as hydrogen or carbon monoxide, or a reduction method using a liquid reducing agent such as ethanol, methanol, hydrazine or sodium borohydride. Further, by the liquid phase reduction method, it is also possible to carry out the supporting of the metal compound containing Pt on the composite oxide support such as a zeolite support and the reduction at the same time, whereby it is also possible to obtain a deodorizing catalyst on which a metal containing Pt is supported. Specifically, the liquid phase reduction can be performed by, for example, adding a platinum diamino dinitrite ethanol solution, citric acid, and ethanol to a composite oxide support such as zeolite, and heating and refluxing the mixture.

Supporting and reduction of the metal compound containing a Pt compound may be performed in one operation or may be performed repeatedly a plurality of times.

<Deodorant composition>

The deodorizing catalyst according to the present invention may be used alone or in combination with other components. For example, it can be used as a deodorant composition containing the deodorizing catalyst according to the present invention and another deodorizing component that removes, neutralizes or decomposes odor components.

As the other deodorizing component, a known component can be used without limitation, but a chemical adsorbent is preferably used. In particular, by mixing a chemical adsorbent which deodorizes by a chemical interaction with an odorant with the deodorizing catalyst according to the present invention described above, a deodorant composition having particularly improved initial performance can be obtained. Examples of the chemical adsorbent include KESMON NS-750 (organic amine-supported silica; for aldehydes), NS-70 (Ca, Mg-based compounds; for acids), NS-10 (zirconium phosphate; for ammonia), and NS-20C (Cu-based compound-supported silica; for sulfur compounds), all of which are manufactured by Toagosei Co., Ltd.; CHEMCATCH (adipic dihydrazide; for aldehydes), which is manufactured by Otsuka Chemical Co., Ltd.; and DUSHLIGHT S (amine compound-supported silica; for aldehydes), which is manufactured by Sinanen Zeomic Co., Ltd.

The deodorant composition of the present invention can be suitably used in uses in which deodorization is required and in uses in which antibacterial properties and antiviral properties are required.

<Deodorizing product (deodorant)>

The deodorizing catalyst and the deodorant composition of the present invention can be appropriately prepared into, for example, a liquid agent, a solid agent, or a gel-like solid agent by a known method, and can be used for various industrial and household deodorant uses. Examples of the deodorizing liquid agent include a liquid agent dissolved in an appropriate organic solvent such as water or ethanol, a liquid agent emulsified with an appropriate surfactant, and an aerosol deodorant obtained by filling a spray container with these liquid agents together with a propellant. Examples of the deodorizing solid agent include a powder agent mixed with a powdery inorganic substance such as silica or perlite, an agent adsorbed on paper or a porous substance, and an agent kneaded into a synthetic resin such as polyethylene. Examples of the gel-like solid deodorants include those added to natural or synthetic polymer gel bases such as agar, carrageenan and polyethylene glycol. In addition, if necessary, a surfactant, a bactericide, a perfume, or a pigment, may be appropriately added to these dosage forms, for example.

The deodorant of the present invention can be used for household use, for example, for deodorization in rooms, refrigerators, toilets, or garbage boxes and for removal and prevention of body odor, and can be appropriately used for industrial use, for example, for removal of malodor in sewage treatment plants, fish processing plants, fish powder production plants, livestock pens, livestock manure or poultry manure drying plants, or pulp mills.

One useful deodorizing product using the deodorizing catalyst and deodorant composition of the present invention is deodorizing fibers. In this case, the deodorizing fiber may be a deodorizing fiber (1) in which the deodorizing catalyst and the deodorant composition are attached or adhered to the surface of the raw material fiber or a deodorizing fiber (2) in which the deodorizing catalyst and the deodorant composition are embedded so as to be exposed on the surface of the raw material fiber. The raw material fiber may be either a natural fiber or a synthetic fiber, and may be any of a short fiber, a long fiber, or a composite fiber having a core-sheath structure, for example. The deodorizing fiber (1) can be obtained by attaching a deodorant-containing liquid composition (coating solution) composed of an aqueous or organic solvent-based suspension containing a deodorant composition to the surface of the raw material fiber by a method such as gravure coating, dipping, or spray coating, and removing a medium such as a solvent.

This deodorant-containing liquid composition (coating solution) may be blended with a binder for improving the adhesion of the deodorant composition to the raw material fiber surface. Examples of the binder include an acrylic binder, an acrylic silicone binder, a styrene binder, and an acrylic styrene binder. The ratio of the deodorant to the binder (deodorant/binder) is usually 95/5 to 10/90, preferably 90/10 to 30/70, and more preferably 80/20 to 40/60, and an appropriate ratio can be selected depending on the uses. The lower the binder ratio, the better the performance of the deodorant, but the deodorant tends to be easily removed from the fibers.

When the deodorant-containing liquid composition has a low viscosity and the deodorant precipitates, a thickeners may be added. Examples of the thickener include polysaccharides such as sodium alginate, methyl cellulose, hydroxypropyl cellulose, and xanthan gum, polyvinyl alcohol, and polymethacrylic acid thickeners. The amount added to the coating solution is 0.01 to 10%, preferably 0.1 to 5%, more preferably 0.2% to 2%. When the wettability of the coating solution to the fibers is insufficient, a surfactant may be added. Examples of the surfactant include sodium alkylbenzenesulfonate, sodium alkyloxybenzenesulfonate, sodium alkylsulfate such as sodium lauryl sulfate, and an acetylene glycol surfactant. The amount added to the coating solution is 0.01 to 10%, preferably 0.1 to 5%, more preferably 0.2% to 2%. The pH of the aqueous coating solution containing the deodorant composition is not particularly limited, but in order to sufficiently exhibit the performance of the deodorant composition, the pH is preferably around 6 to 8.

The deodorizing fiber (2) can also be obtained by blending the deodorant composition of the present invention with a melt of a liquid resin for fiber or a solution of a dissolved resin for fiber, and fiberizing the obtained deodorant-containing resin composition. The resin for fiber that can be used in this method is not particularly limited, and any known chemical fibers can be used. Preferred resins include polyester, polyamide, acrylic, polyethylene, polypropylene, polyvinyl, polyvinylidene, polyurethane, and polystyrene. These resins may be a homopolymer or a copolymer. In the case of a copolymer, the polymerization ratio of monomers is not particularly limited.

The ratio of the deodorizing catalyst and the deodorant composition contained in the deodorant-containing resin composition is not particularly limited. In general, when the content of the deodorizing catalyst and the deodorant composition is increased, the deodorizing property can be strongly exhibited and can be maintained for a long period of time; but when the deodorizing catalyst and the deodorant composition are contained in a certain amount or more, the deodorant effect may be not significantly different or the strength of the deodorizing fiber may decrease, and thus the content is preferably 0.1 to 20 parts by mass and more preferably 0.5 to 10 parts by mass with respect to 100 parts by mass of the fiber resin.

Another major deodorizing product using the deodorizing catalyst and deodorant composition of the present invention is deodorant-containing coating compositions. In the production of the deodorant-containing coating composition, the fats and oils or resins as the main component of the coating vehicle used are not particularly limited, and may be any of natural vegetable oils, natural resins, semi-synthetic resins and synthetic resins. Examples of usable fats and oils and resins include drying oils or semi-drying oils such as linseed oil, tung oil and soybean oil; rosin, nitrocellulose, ethyl cellulose, cellulose butyrate, benzyl cellulose, novolak type or resol type phenol resins, alkyd resins, aminoalkyd resins, acrylic resins, vinyl chloride resins, silicone resins, fluorine resins, epoxy resins, urethane resins, saturated polyester resins, melamine resins, and polyvinylidene chloride resins. The deodorant-containing coating composition may be thermoplastic or curable.

The ratio of the deodorizing catalyst and the deodorant composition of the present invention contained in the deodorant-containing coating composition is not particularly limited. In general, when the content of the deodorizing catalyst and the deodorant composition is increased, the deodorizing property can be strongly exhibited and can be maintained for a long period of time; but when the deodorizing catalyst and the deodorant composition are contained in a certain amount or more, the deodorant effect may be not significantly different or the coating surface loses gloss or cracks. Therefore, the content proportion of the deodorizing catalyst and deodorant composition is preferably 0.1 to 20% by mass, more preferably 0.5 to 10% by mass, based on 100% by mass of the composition.

The deodorizing catalyst and deodorant composition of the present invention can be used for both liquid coating materials and powder coating materials. The deodorant-containing coating composition may be of a type that forms a film by any mechanism, and in the case of curing the coating film, it may be of an oxidative polymerization type, a moisture polymerization type, a heat curing type, a catalyst curing type, an ultraviolet curing type, or a polyol curing type, for example. It can also be applied to a sintered coating that cures by baking. Further, the pigment, dispersant and other additives to be blended in the composition are not particularly limited except for those having a possibility of causing a chemical reaction with the deodorizing catalyst and deodorant composition of the present invention. The deodorant-containing coating composition can be easily prepared, and specifically, the raw material components may be sufficiently dispersed and mixed using a general mixing apparatus such as a ball mill, a roll mill, a disperser, or a mixer.

Still another deodorizing product using the deodorizing catalyst and the deodorant composition of the present invention is a deodorizing sheet (including a deodorizing film). The raw material sheet before processing is not particularly limited, and the material or the fine structure thereof, for example, can be set in accordance with the intended use, for example. Preferred materials for the raw material sheet are organic materials such as resin and paper, inorganic materials, or composites thereof. Those having air permeability from one surface side to the other surface side are preferably used as the raw material sheet. Other preferable specific examples of the raw material sheet include Japanese paper, synthetic paper, nonwoven fabric, and resin film, and a particularly preferable raw material sheet is paper made of natural pulp and/or synthetic pulp. When natural pulp is used, the deodorant particles are easily sandwiched between the finely branched fibers, and a practical carrier can be obtained especially without the use of binders. On the other hand, the synthetic pulp has an advantage of excellent chemical resistance. In the case of using the synthetic pulp, since it may be difficult to support the deodorant particles by sandwiching the powder between the fibers, in order to suppress this, a part of the fibers may be melted in the drying step after the papermaking to increase the adhesion between the powder and the fibers, or another thermosetting resin fiber may be mixed in a part of the fibers. When natural pulp and synthetic pulp are mixed and used in appropriate proportions, paper with various adjusted properties can be obtained, but generally, when the proportion of synthetic pulp is increased, paper with excellent strength, water resistance, chemical resistance, and oil resistance, for example, can be obtained, while when the proportion of natural pulp is increased, paper with excellent water absorption, gas permeability, hydrophilicity, moldability, and texture, for example, can be obtained.

The deodorizing sheet may contain the deodorant composition from one side of the raw material sheet to the other side as a whole, may be arranged on the surface layer on one or the other side, or may be arranged inside the material sheet except the surface layer.

The loadings of the deodorizing catalyst and deodorant composition of the present invention in the deodorizing sheet are not particularly limited. In general, when the loading of the deodorizing catalyst and the deodorant composition is increased, the deodorizing property can be strongly exhibited and can be maintained for a long period of time; but when the deodorizing catalyst and the deodorant composition are supported in a certain amount or more, the deodorant effect is not significantly different. Therefore, the loading of the deodorizing catalyst and the deodorant composition is preferably 0.1 to 10 parts by mass per 100 parts by mass of the raw material sheet.

The method for producing the deodorizing sheet is not particularly limited. The deodorizing catalyst and the deodorant composition of the present invention may be supported at the same time as the production of the raw material sheet or after the production of the raw material sheet. For example, in the case of supporting on paper, a method of introducing the deodorizing catalyst and the deodorant composition in any step of the papermaking process or a method of applying, immersing or spraying a deodorant-containing liquid composition (coating solution) containing a binder to paper produced in advance can be applied. The kind of the binder, and the thickening agent and the surfactant which can be added to the coating solution are the same as those described in the section of the deodorizing fiber. In the case of using the deodorant-containing liquid composition, it is preferable to apply the liquid composition so that the loading of the deodorizing catalyst and the deodorant composition is about 0.05 to 10 $g/m^2$.

<Method for removing odor>

The method for removing an odor of the present invention is a method for reducing and removing an odor of an odorant by using the deodorizing catalyst of the present invention or a deodorant composition or deodorizing product containing the same (hereinafter, may also be referred to as deodorizing catalyst), preferably a method for reducing an odor of at least one odorant selected from aldehydes, carboxylic acids, esters, amines, thiols, and hydrocarbons. The deodorizing catalyst may be used as it is, or may be used in combination with other materials.

The odor removal method of the present invention can be achieved by bringing the deodorizing catalyst of the present invention, etc. described above into contact with a gas to be subjected to odor removal (a gas containing an odorant), and specific examples thereof include a method in which the deodorizing catalyst, etc. of the present invention is placed in a space to be subjected to odor removal, and a method in which a gas containing an odorant is passed through a space where the deodorizing catalyst, etc. of the present invention is present.

When odor is removed using the deodorizing catalyst, etc. according to the present invention, the composite oxide such as zeolite constituting the deodorizing catalyst, etc. has a high initial odor reduction effect because it adsorbs surrounding odorants, and the adsorbed odorants are decomposed by the supported metal component containing Pt, so that the adsorbed odorants are not released and the adsorption ability is recovered, whereby the odor caused by the odorants can be reduced and removed over a long period of time. Further, when a deodorant composition or a deodorizing product containing a chemical adsorbent that deodorizes by chemical interaction with an odorant is used together with the deodorizing catalyst according to the present invention, the initial odor reduction effect can be further improved.

<Uses>

As described above, the deodorizing catalyst of the present invention and the deodorant composition and deodorizing product containing the same can reduce and remove odors over a long period of time by adsorbing and decomposing surrounding odorants, and in particular, can reduce and remove odors caused by odorants selected from aldehydes, carboxylic acids, esters, amines, thiols, and hydrocarbons. Further, since the deodorizing catalyst of the present invention and the deodorant composition and the deodorizing product containing the same has antibacterial/antiviral properties, and is particularly excellent in antibacterial properties, it can be suitably used in uses where antibacterial/antiviral properties are required for the surface of a product, for example.

Such a deodorizing catalyst of the present invention and deodorant composition containing the same can be suitably used for reduction and removal of an odor caused by odorants in the air, and can be particularly suitably used for, for example, deodorization in a room such as an automobile.

Further, the deodorizing catalyst, the deodorant composition, and the deodorizing product of the present invention can be applied to various uses without particular limitation, and can be applied to, for example, a upholstery material, a curtain, a carpet, a tile, a wallpaper, or an interior material for a vehicle. For these uses, any form of the above deodorizing product may be used.

Examples of the vehicles in which the interiors for a vehicle is used include an automobile, a train, a passenger plane, and a ship.

Examples of the interior material for a vehicle include a ceiling material, an inner panel, a door trim, a headrest, a steering wheel, a shift lever, an instrument panel, a seat cover, a floor material, a floor mat, and a rear door panel.

As other uses of the deodorizing product of the present invention, in particular, the deodorizing fiber can be used for, for example, fiber products such as products obtained by processing a deodorizing sheet described below, for example, clothes such as underwear, socks, and aprons, nursing clothes, bedclothes, a cushion, a blanket, a carpet, a sofa, an air filter, a quilt cover, a curtain, and a car sheet.

As other uses of the deodorizing product of the present invention, in particular, the deodorizing coating composition can be suitably used for, for example, inner walls and outer walls of buildings, vehicles, and railroad vehicles; and facilities of garbage incineration plants; and garbage containers. In particular, it can be used for building materials such as floor materials and inner walls of factories with high VOC concentrations, and floor materials and inner walls of fish markets.

Since the deodorizing catalyst of the present invention and the deodorant composition and the deodorizing product containing the same have antibacterial/antiviral properties, they can be used as antibacterial/antiviral deodorizing products. Specific uses are the same as those described above, and examples thereof include an upholstery material, a curtain, a carpet, a tile, a wallpaper, or an interior material for a vehicle.

EXAMPLES

The present invention will be described in more detail below based on Examples, but the present invention is not limited to these Examples.

[Example 1]

Preparation of deodorizing catalyst A

As a compound containing Pt, 0.05 g of hydrogen hexachloroplatinate (IV) hexahydrate (manufactured by FUJIFILM Wako Pure Chemical Corporation) was dissolved in 50 cc of distilled water. The solution was fed into a 200 cc eggplant flask and then 2.0 g of MFI type zeolite (H-ZSM-5, HSZ 822HOA manufactured by Tosoh Corporation, silica/alumina ratio ($SiO_2/Al_2O_3$ ratio (mol/mol)): 23, average particle size: 5 $\mu$m) was fed to impregnate the zeolite with a Pt compound solution to yield 1% Pt/zeolite. The flask was then attached to an evaporator device, water was evaporated under vacuum at 80°C, and the remaining powder was recovered. The recovered powder was heated to 120°C at a rate of 5°C/min in a 100% $H_2$ flow and hydrogen-reduced for 2 hours to prepare a deodorizing catalyst A, which is a Pt/zeolite catalyst.

Evaluation of odor removal degree (propionaldehyde)

Into a 2L sampling bag (manufactured by OMI ODOR AIR SERVICE Co.,Ltd.), Flek-Sampler, 2 mg of the deodorizing catalyst A obtained above was placed. In the case of a blank test, no catalyst is used.

1.5 L of air through an activated carbon filter is introduced. Then, propionaldehyde is introduced so that the concentration thereof in the sampling bag becomes 1 ppm.

After being allowed to stand at room temperature for 24 hours, the odor was evaluated using an odor sensor (manufactured by SHINYEI Technology Co., LTD., model number: OMX-TDM), and the degree of odor removal was calculated by the following formula. The results are shown in Table 1.

```
Degree of odor removal (%) = (1 - sensor value (with

catalyst) / sensor value (blank)) × 100
```

Evaluation of degree of reduction

0.2 g of the deodorizing catalyst A obtained above was fixed in a sample tube. The sample tube was attached to a catalyst analyzer (catalyst analyzer BELCAT-A, manufactured by BEL Japan, Inc.) equipped with a thermal conductivity detector (TCD), treated at 120°C for 2 hours under He flow to remove adsorbed water, and cooled to 40°C under He flow. Next, the gas was analyzed with a thermal conductivity detector (TCD) while raising the temperature at 5°C/ min under a hydrogen flow, and the peak area X when the temperature was raised from 40°C to 400°C for reduction was measured.

Meanwhile, 0.2g of the prepared deodorizing catalyst was fixed in a sample tube. The sample tube was attached to a catalyst analyzer (catalyst analyzer BELCAT-A, manufactured by BEL Japan, Inc.) equipped with a thermal conductivity detector (TCD), treated at 150°C for 2 hours under oxygen flow to completely oxidize the supported metal component, and cooled to room temperature under He flow to obtain an oxidation catalyst. Next, the gas was analyzed with a thermal conductivity detector (TCD) while raising the temperature at 5°C/ min under a hydrogen flow, and the peak area Y when the temperature was raised from 40°C to 400°C for reduction was measured.

From the obtained peak areas X and Y, the degree of reduction was determined by the following formula (1). The results are shown in Table 1.

$$\text{degree of reduction (\%)} = 100 - [X/Y] \times 100 \qquad (1)$$

(In the formula (1), X represents a peak area obtained from a thermal conductivity detector (TCD) when the catalyst is dried and reduced by heating from 40°C to 400°C, and Y represents a peak area obtained from the TCD when an oxidation catalyst obtained by heat-treating the catalyst in an air or under oxygen atmosphere at 150°C for 2 hours and then cooling the catalyst to 40°C or lower is reduced by heating from 40°C to 400°C.)

[Examples 2 and 3 and Comparative Examples 1 and 2]

Preparation of deodorizing catalysts B, C, D, and E

Deodorizing catalysts B, C, D, and E, which are Pt/zeolite catalysts, were prepared in the same manner as in Example 1, except that the heating temperature (reduction temperature) during hydrogen reduction was changed to the temperature shown in Table 1. The degree of odor removal and the degree of reduction were determined in the same manner as in Example 1 for each of the obtained deodorizing catalysts. The results are shown in Table 1.

[Table 1]

Table 1

| | Deodorizing catalyst | Reduction temperature during catalyst preparation | Degree of reduction | Degree of odor removal (propionaldehyde) |
|---|---|---|---|---|
| Example 1 | A | 120°C | 62 % | 51 % |
| Example 2 | B | 150°C | 71 % | 58 % |
| Example 3 | C | 250°C | 86 % | 62 % |
| Comparative Example 1 | D | 80°C | 57 % | 36 % |
| Comparative Example 2 | E | 300°C | 88 % | 43 % |

From Examples 1 to 3 and Comparative Examples 1 and 2, it was found that the deodorizing catalyst supporting Pt and having a degree of reduction within the specific range of 60% to 86% exhibited an excellent odor removal effect.

[Example 4]

Continuous decomposition test of propionaldehyde

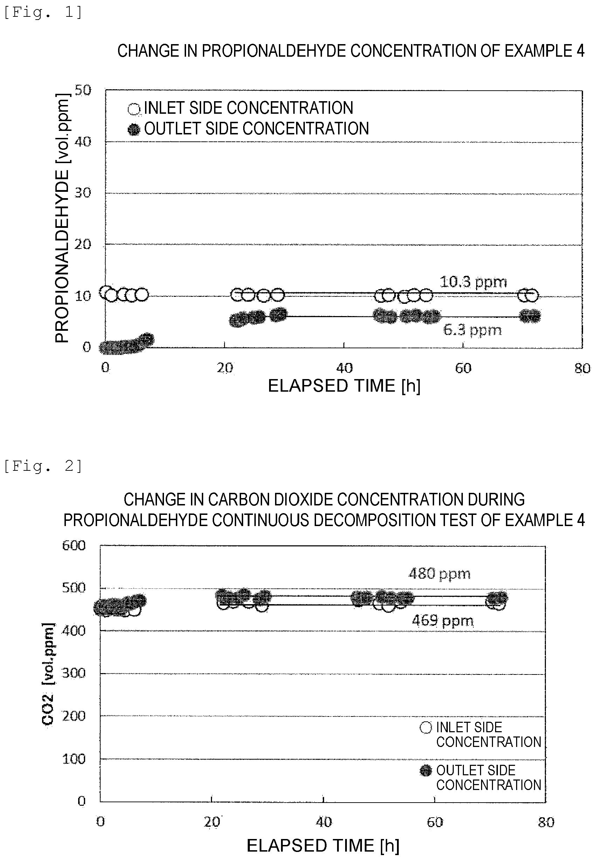

A continuous decomposition test in which a gas containing propionaldehyde as an odorant was continuously treated in a fixed bed flow system using 10 mg of the deodorizing catalysts B obtained in Example 2 was carried out as follows.
In a reaction tube, 10 mg of the deodorizing catalyst B was placed, and 10 ppm of propionaldehyde gas mixture (diluted with air) was passed at 3 L/h at 80°C. The reaction tube inlet gas and outlet gas were introduced into GC-MS for analysis of propionaldehyde and carbon dioxide concentrations.
From the analysis results, Figure 1 shows graphs showing changes over time in propionaldehyde concentration, and Figure 2 shows graphs showing changes over time in carbon dioxide concentration during the propionaldehyde continuous decomposition test. From Figure 1, it was found that the propionaldehyde concentration steadily decreased, and from Figure 2, it was found that carbon dioxide was continuously generated, and it was found that propionaldehyde (aldehydes), which is an odorant, can be continuously removed for a long period of time by the deodorizing catalyst B.

[Example 5]

Continuous decomposition test of ethyl acetate

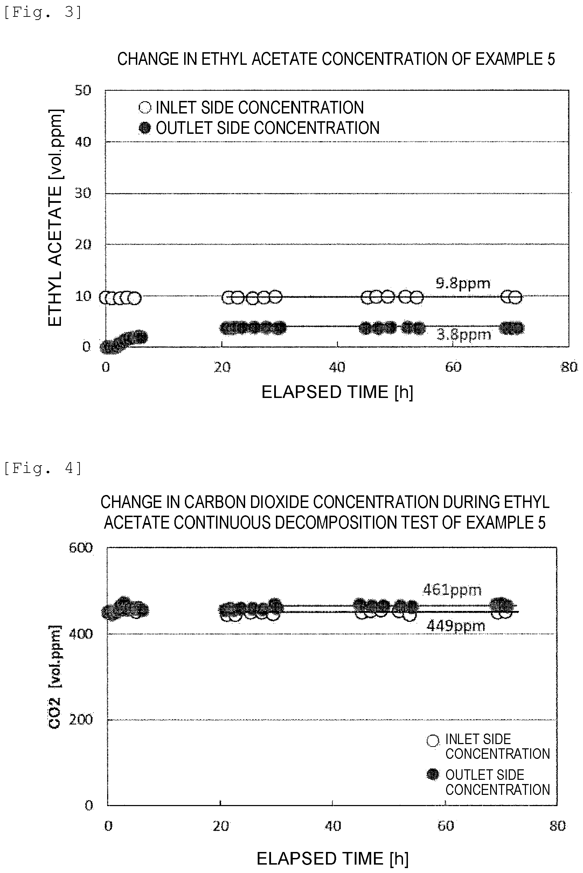

A continuous decomposition test was performed in the same manner as in Example 4, except that 10 ppm of ethyl acetate gas mixture (diluted with air) was used instead of 10 ppm of propionaldehyde gas mixture in Example 4, and the ethyl acetate concentration and the carbon dioxide concentration of the reaction tube inlet gas and outlet gas were analyzed.
From the analysis results, Figure 3 shows graphs showing changes over time in ethyl acetate concentration, and Figure 4 shows graphs showing changes over time in carbon dioxide concentration during the continuous decomposition test. From Figure 3, it was found that the ethyl acetate concentration steadily decreased, and from Figure 4, it was found that carbon dioxide was continuously generated, and it was found that ethyl acetate (carboxylic acids), which is an odorant, can be continuously removed for a long period of time by the deodorizing catalyst B.

[Example 6]

Continuous decomposition test of trimethylamine

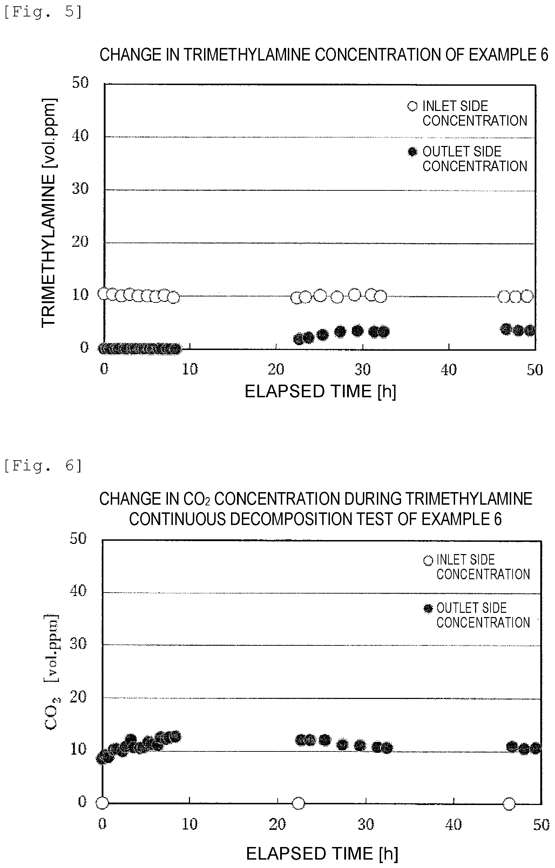

A continuous decomposition test was performed in the same manner as in Example 4, except that 10 ppm of trimethylamine gas mixture (diluted with air) was used instead of 10 ppm of propionaldehyde gas mixture in Example 4 and the temperature was set to 120°C, and the trimethylamine concentration and the carbon dioxide concentration of the reaction tube inlet gas and outlet gas were analyzed.
From the analysis results, Figure 5 shows graphs showing changes over time in trimethylamine concentration, and Figure 6 shows graphs showing changes over time in carbon dioxide concentration during the continuous decomposition test. From Figure 5, it was found that the ethyl acetate concentration steadily decreased, and from Figure 6, it was found that carbon dioxide was continuously generated, and it was found that trimethylamine (amines), which is an odorant, can be continuously removed for a long period of time by the deodorizing catalyst B.

[Example 7]

Continuous decomposition test of methyl mercaptan

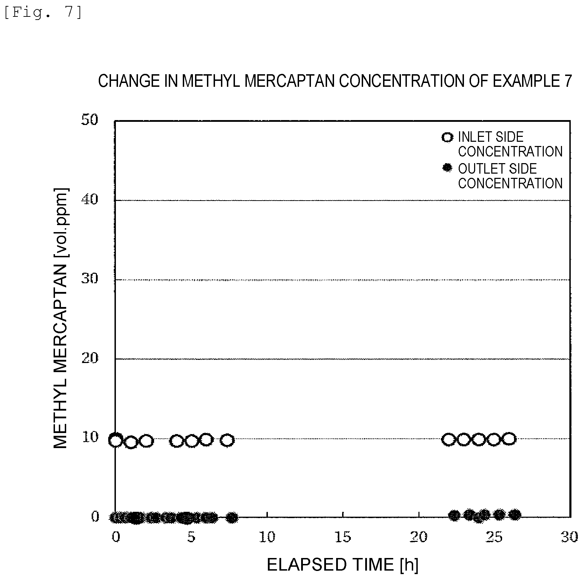

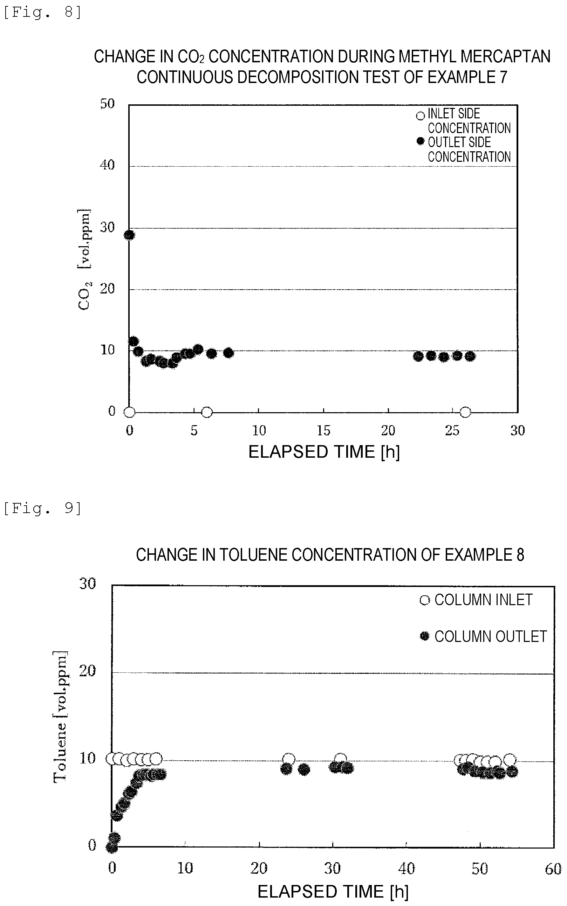

A continuous decomposition test was performed in the same manner as in Example 4, except that 10 ppm of methyl mercaptan gas mixture (diluted with air) was used instead of 10 ppm of propionaldehyde gas mixture in Example 4 and the temperature was set to 160°C, and the methyl mercaptan concentration and the carbon dioxide concentration of the reaction tube inlet gas and outlet gas were analyzed.
From the analysis results, Figure 7 shows graphs showing changes over time in methyl mercaptan concentration, and Figure 8 shows graphs showing changes over time in carbon dioxide concentration during the continuous decomposition test. From Figure 7, it was found that the ethyl acetate concentration steadily decreased, and from Figure 8, it was found that carbon dioxide was continuously generated, and it was found that methyl mercaptan (sulfur compound), which is an odorant, can be continuously removed for a long period of time by the deodorizing catalyst B.

[Example 8]

Continuous decomposition test of toluene

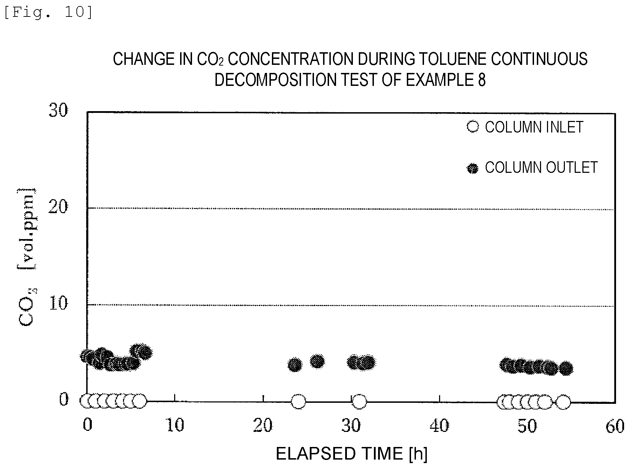

A continuous decomposition test was performed in the same manner as in Example 4, except that 10 ppm of toluene gas mixture (diluted with air) was used instead of 10 ppm of propionaldehyde gas mixture in Example 4, and the toluene concentration and the carbon dioxide concentration of the reaction tube inlet gas and outlet gas were analyzed.
From the analysis results, Figure 9 shows graphs showing changes over time in toluene concentration, and Figure 10 shows graphs showing changes over time in carbon dioxide concentration during the continuous decomposition test. From Figure 9, it was found that the toluene concentration steadily decreased, and from Figure 10, it was found that carbon dioxide was continuously generated, and it was found that toluene (aromatic hydrocarbons), which is an odorant, can be continuously removed for a long period of time by the deodorizing catalyst B.

[Example 9]

Mixed odorant decomposition test

In a 500 ml headspace bottle, 10 mg of the deodorizing catalyst B obtained in Example 2 was placed, which was then sealed in an air atmosphere, and 10 ml of the internal gas was collected and exhausted. Here, 10 ml of a gas mixture containing 12,500 vol.ppm of each odorant (propionaldehyde, ethyl acetate, n-butyric acid) was injected (the initial concentration of each odorant in the container was 250 ppm). The time of injection was set to 0 h, and the container was allowed to stand in an air thermostat at 80°C. After 2, 4, 6, 22, 24, 26, 28, and 30 hours, sampling and addition of fresh gas were performed by the following steps 1 to 3:

(step 1) from the reaction container, 1 ml of the internal gas was collected and subjected to GC-MS analysis under the conditions shown in Table 2 below;
(step 2) from the reaction container, 9 ml of internal gas was collected and exhausted; and
(step 3) 10 ml of odorant mixed gas was added thereto.

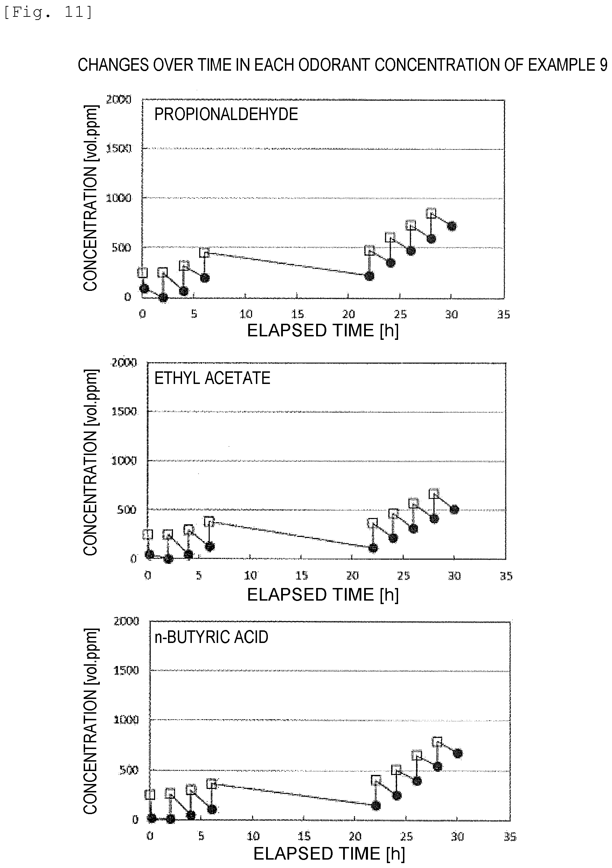

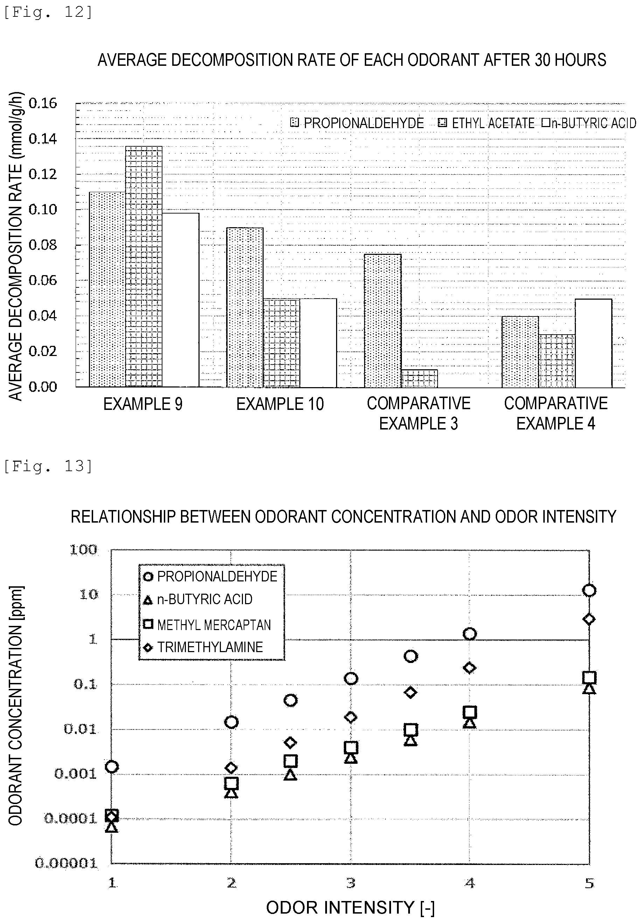

The concentration of each odorant immediately after the addition of the mixed gas was obtained from a calculated value. Figure 11 shows graphs of changes over time in each odorant concentration. In each graph of Figure 11, a circle (•) represents each odorant concentration (GC-MS analysis value) obtained by sampling in the step 1, and a square (□) represents each odorant concentration (calculation value) immediately after the addition of the odorant mixed gas in the step 3. Also, from the change in concentration of each odorant, the average decomposition rate (mmol/g/h) of each odorant per 1 g of the catalyst after 30 hours was determined and shown in the graphs of Figure 12. The values in the graphs of Figure 12 indicate, from the left, the average decomposition rate of propionaldehyde, the average decomposition rate of ethyl acetate, and the average decomposition rate of n-butyric acid, respectively.

[Table 2]

Table 2

| GC-MS analysis condition | |
|---|---|
| Equipment | GC system 7890A (Agilent Technologies) JMS-QI000GC MKII Ultra Quad GC/MS (JEOL) |
| Column | Stabilwax-MS (manufactured by Restec) I.D.0.25mm×30m, d=0.25um |
| Carrier gas | He 1ml/min |
| Temperature conditions | injection site: 200°C Column: 40°C-1 min hold 40→60°C, 5°C/min heating 60→240°C, 10°C/min heating interface: 240°C |

(continued)

| GC-MS analysis condition | |
|---|---|
| Amount injected | 1 ml<br>split ratio 1:10 |
| Ionization condition | ionization method: EI (electron impact)<br>ionization energy: 70eV<br>ionization source temperature: 200°C |

[Example 10]

Coated film formation

By mixing and stirring 6 g of the deodorizing catalyst B obtained in Example 2, 10 g of 5% sodium alginate, 40 g of acrylic silicone binder light epoch S-65 (manufactured by Hokko Chemical Co., Ltd., solid content 25%) and 44 g of water, an aqueous solution for coating in which particles were uniformly dispersed was obtained. The aqueous coating solution was applied onto a PET film (manufactured by TOYOBO CO., LTD., thickness: 25 $\mu$m) using an automatic film applicator (manufactured by TESTER SANGYO CO., LTD.), and then dried at 130°C for 15 minutes to obtain a coated film in which particles were uniformly dispersed. The coating mass was 2.5 g/m$^2$.

Mixed odorant decomposition test

The same operation as in Example 9 was performed except that the coated film 4 $\times$ 10 cm obtained above (corresponding to 10 mg of deodorizing catalyst B) was used instead of the deodorizing catalyst B in Example 9. The average decomposition rate (mmol/g/h) of each odorant per 1 g of the catalyst after 30 hours was determined and shown in the graphs of Figure 12.

[Comparative Example 3]

The same operation as in Example 9 was performed except that 10 mg of coconut shell activated carbon (manufactured by NACALAI TESQUE INC.) was used instead of the deodorizing catalyst B in Example 9. The average decomposition rate (mmol/g/h) of each odorant per 1 g of the catalyst after 30 hours was determined and shown in the graphs of Figure 12.

[Comparative Example 4]

The same operation as in Example 9 was performed except that 10 mg of MFI type zeolite (H-ZSM-5, HSZ 822HOA manufactured by Tosoh Corporation, silica/alumina ratio (SiO$_2$/Al$_2$O$_3$ ratio (mol/mol)): 23, average particle size: 5 $\mu$m) was used instead of the deodorizing catalyst B in Example 9. The average decomposition rate (mmol/g/h) of each odorant per 1 g of the catalyst after 30 hours was determined and shown in the graphs of Figure 12.

[Example 11]

Deodorant composition containing chemical adsorbent

Into a 2L sampling bag (Fleck sampler, manufactured by OMI ODOR AIR SERVICE Co.,Ltd.), 5 mg of the deodorizing catalyst B obtained in Example 2, 2.5 mg of KESMON NS-750 (manufactured by Toagosei Co., Ltd.) and 2.5 mg of KESMON NS-70 (manufactured by Toagosei Co., Ltd.) serving as chemical adsorbent were added as a deodorant composition. Air through an activated carbon filter was introduced into this 2 L sampling bag, and a silicone cap was placed on the inlet. Then, 1 mL of propionaldehyde/air at 1500 vol.ppm was injected into the sampling bag using a gas-tight syringe to adjust the propionaldehyde concentration in the bag to about 1 ppm. The odor intensity was sensory evaluated at room temperature for 1 hour, 5 hours, and 24 hours stand. Here, the odor intensity is an index obtained by quantifying the degree of odor as shown in Table 3 below. The indices in this sensory evaluation were also used in the following Examples and Comparative Examples. In the sensory evaluation, samples of each odor intensity were prepared from the relationship between propionaldehyde concentration and odor intensity as shown in the graph (Figure 13) based on the relationship between the concentration of each odorant and odor intensity (http://kjknpo.com/html#j/bukai/kuki/qa/a11.htm) by the Air Environment Committee of Japan Health Housing Association, and were evaluated by comparison with them. The evaluation results are shown in Table 4.

[Table 3]

Table 3 Sensory evaluation index

| Odor intensity | Odor strength |
|---|---|
| 0 | Not perceptible |
| 1 | Barely perceptible |
| 2 | Faint but identifiable |
| 3 | Easily perceptible |
| 4 | Strong |
| 5 | Very strong |

[Comparative Example 5]

The same operation as in Example 11 was performed except that the deodorant composition was changed to 10 mg of coconut shell activated carbon (manufactured by NACALAI TESQUE INC.), and the sensory evaluation results are shown in Table 4.

[Comparative Example 6]

The same operation as in Example 11 was performed except that no deodorant composition was used, and the sensory evaluation results are shown in Table 4.

[Table 4]

Table 4

| | Deodorant composition | Sensory evaluation results | | |
|---|---|---|---|---|
| | | 1 h | 5 h | 24 h |
| Example 11 | Deodorizing catalyst B 5 mg +KESMON NS-750 2.5 mg +KESMON NS-70 2.5 mg | 1.5 | 0.5 | 0 |
| Comparative Example 5 | coconut shell activated carbon 10 mg | 1.5 | 0.5 | 0.5 |
| Comparative Example 6 | none | 4 | 4 | 4 |

[Example 12]

Odor return evaluation due to temperature rise

Into a 2L sampling bag (Fleck sampler, manufactured by OMI ODOR AIR SERVICE Co.,Ltd.), 25 mg of the deodorizing catalyst B obtained in Example 2, 12.5 mg of KESMON NS-750, and 12.5 mg of KESMON NS-70 were added as a deodorant composition. Air through an activated carbon filter was introduced into a 2.0 L sampling bag, and a silicone cap was placed on the inlet. Then, 1 mL of propionaldehyde/air at 1500 vol.ppm was injected into the sampling bag using a gas-tight syringe to adjust the propionaldehyde concentration in the bag to about 1 ppm. At room temperature, the odor intensity was zero when the product was allowed to stand for two days. The test bag was placed in an oven as it was, heated to 40°C, left to stand for 1 hour, and then subjected to sensory evaluation. Thereafter, the same evaluation was performed at 60°C and 80°C. The results are shown in Table 5.

[Comparative Example 7]

The same operation as in Example 12 was performed except that the deodorant composition was changed to 50 mg of coconut shell activated carbon (manufactured by NACALAI TESQUE INC.) in Example 12, and sensory evaluation was performed. The results are shown in Table 5.

[Comparative Example 8]

The same operation as in Example 12 was performed except that the deodorant composition was not used in Example 12, and sensory evaluation was performed. The results are shown in Table 5.

[Table 5]

Table 5

| | Deodorant composition | Sensory evaluation results | | |
|---|---|---|---|---|
| | | 40°C ×1 h | 60°C ×1 h | 80°C ×1 h |
| Example 12 | Deodorizing catalyst B 25 mg +KESMON NS-750 12.5 mg+KESMON NS-70 12.5 mg | 0 | 0 | 0 |
| Comparative Example 7 | coconut shell activated carbon 50 mg | 0 | 0.5 | 2 |
| Comparative Example 8 | none | 3 | 3 | 3.5 |

[Example 13]

(Combination of deodorizing catalyst and chemical adsorbent; butyric acid)

Into a 2L sampling bag (Fleck sampler, manufactured by OMI ODOR AIR SERVICE Co.,Ltd.), 5 mg of the deodorizing catalyst B obtained in Example 2, 2.5 mg of KESMON NS-750 (manufactured by Toagosei Co., Ltd.) and 2.5 mg of KESMON NS-70 (manufactured by Toagosei Co., Ltd.) serving as chemical adsorbent were added as a deodorant composition. Air through an activated carbon filter was introduced into this 2 L sampling bag, and a silicone cap was placed on the inlet. Then, 1 mL of n-butyric acid/air at 1500 vol.ppm was injected into the sampling bag using a gas-tight syringe to adjust the n-butyric acid concentration in the bag to about 1 ppm. The odor intensity was sensory evaluated at room temperature for 1 hour, 5 hours, and 24 hours stand. The odor intensity is an index obtained by quantifying the degree of odor as shown in Table 3. Samples of each odor intensity were prepared from the relationship between the concentration of n-butyric acid and the odor intensity shown in the graphs of Figure 13, and the sensory evaluation was performed by comparison with the samples. The evaluation results are shown in Table 6.

[Comparative Example 9]

The same operation as in Example 13 was performed except that the deodorant composition was changed to 10 mg of coconut shell activated carbon (manufactured by NACALAI TESQUE INC.) in Example 14, and sensory evaluation was performed. The results are shown in Table 6.

[Comparative Example 10]

The same operation as in Example 13 was performed except that the deodorant composition was not used in Example 13, and sensory evaluation was performed. The results are shown in Table 6.

[Table 6]

Table 6

|  | Deodorant composition | Sensory evaluation results | | |
|---|---|---|---|---|
|  |  | 1 h | 5 h | 24 h |
| Example 13 | Deodorizing catalyst B 5 mg +KESMON NS-750 2.5 mg+KESMON NS-70 2.5 mg | 0 | 0 | 0 |
| Comparative Example 9 | coconut shell activated carbon 10 mg | 2.5 | 1.0 | 0.5 |
| Comparative Example 10 | none | 4 | 4 | 3.5 |

[Example 14]

(Combination of deodorizing catalyst and chemical adsorbent; trimethylamine)

Into a 2L sampling bag (Fleck sampler, manufactured by OMI ODOR AIR SERVICE Co.,Ltd.), 5 mg of the deodorizing catalyst B obtained in Example 2, 2.5 mg of KESMON NS-750 (manufactured by Toagosei Co., Ltd.) and 2.5 mg of KESMON NS-70 (manufactured by Toagosei Co., Ltd.) serving as chemical adsorbent were added as a deodorant composition. Air through an activated carbon filter was introduced into this 2 L sampling bag, and a silicone cap was placed on the inlet. Then, 1 mL of trimethylamine/air at 1500 vol.ppm was injected into the sampling bag using a gas-tight syringe to adjust the trimethylamine concentration in the bag to about 1 ppm. The odor intensity was sensory evaluated at room temperature for 1 hour, 5 hours, and 24 hours. The odor intensity is an index obtained by quantifying the odor strength as shown in Table 3. Samples of each odor intensity were prepared from the relationship between the concentration of trimethylamine and the odor intensity shown in the graphs of Figure 7, and the sensory evaluation was performed by comparison with the samples. The evaluation results are shown in Table 7.

[Comparative Example 11]

The same operation as in Example 14 was performed except that the deodorant composition was changed to 10 mg of coconut shell activated carbon (manufactured by NACALAI TESQUE INC.) in Example 14, and sensory evaluation was performed. The results are shown in Table 7.

[Comparative Example 12]

The same operation as in Example 15 was performed except that the deodorant composition was not used in Example 14, and sensory evaluation was performed. The results are shown in Table 7.

[Table 7]

Table 7

|  | Deodorant composition | Sensory evaluation results | | |
|---|---|---|---|---|
|  |  | 1 h | 5 h | 24 h |
| Example 14 | Deodorizing catalyst B 5 mg +KESMON NS-750 2.5 mg+KESMON NS-70 2.5 mg | 2.5 | 1 | 0 |
| Comparative Example 11 | coconut shell activated carbon 10 mg | 2.5 | 1.5 | 0.5 |
| Comparative Example 12 | none | 5 | 5 | 4 |

[Example 15]

Odor return evaluation due to temperature rise

A bag containing the deodorant composition obtained after 24 hours in Example 14 was placed in an oven as it was, heated to 80°C, left to stand for 1 hour, and then subjected to sensory evaluation. The odor intensity remained unchanged at 0.

[Comparative Example 13]

Odor return evaluation due to temperature rise

A bag containing the deodorant composition obtained after 24 hours in Comparative Example 11 was placed in an oven as it was, heated to 80°C, left to stand for 1 hour, and then subjected to sensory evaluation. The odor intensity increased from 0.5 to 2.5, confirming that the odor returned by heating.

[Example 16]

Deodorizing performance evaluation (trimethylamine)

Into a 3L sampling bag (Fleck sampler, manufactured by OMI ODOR AIR SERVICE Co.,Ltd.), 3 mg of the deodorizing catalyst B obtained in Example 2 was added, and 2.5 L of air through an activated carbon filter was introduced, and the inlet was capped with silicone. Then, 5.5 mL of trimethylamine/air at 20000 vol.ppm was injected into the sampling bag using a gas-tight syringe to adjust the trimethylamine concentration in the bag to about 45 ppm. The concentration of trimethylamine in the bag was evaluated with a detector tube 180 (manufactured by GASTEC CORPORATION) when the bag was left to stand for 16 hours at 80°C. The same evaluation was performed without adding the deodorizing catalyst as a blank, and the relative concentration was 12% when the value of the blank was 100%.

[Example 17]

Production of binder 1

Into a reaction container equipped with a stirrer, a reflux condenser, a dropping device, and a thermometer, 550 g of ion-exchanged water and 1 g of disodium 4-dodecyl-2,4'-oxydibenzenesulfonate were charged and the temperature was raised to 72°C while nitrogen was replaced under stirring. The internal temperature was maintained at 72°C, 3 g of potassium persulfate was added as a polymerization initiator, and after dissolution, an emulsion previously prepared by adding 15 g of acryl amide, 590 g of methyl methacrylate, 330 g of n-butyl acrylate, 20 g of 2-hydroxyethyl methacrylate, 20 g of methacrylic acid, 15 g of ethylene glycol dimethacrylate, and 1 g of t-dodecylmercaptan to 400 g of ion-exchanged water and 3 g of disodium 4-dodecyl-2,4'-oxydibenzenesulfonate under stirring was continuously added dropwise to the reactant for 4 hours. After completion of dropping, aging was performed for 2 hours. The obtained aqueous solution containing the binder 1 was cooled to room temperature, and then adjusted to a solids content of 50% and pH 7.5 by adding ion-exchanged water and an aqueous ammonium solution to obtain a 50% aqueous solution of binder 1. Coating solution preparation/application (production of coated nonwoven fabric N1)

By mixing and stirring 3 g of the deodorizing catalyst B obtained in Example 2, 4 g of the 50% binder 1 aqueous solution, 6 g of a 5% THICKENER SN615 (manufactured by San Nopco Ltd.) aqueous solution, 2 g of a 10% DOWFAX 2A1 (manufactured by Dow, Inc.) aqueous solution and 32 g of water, a coating solution in which particles were uniformly dispersed was obtained. The obtained coating solution was poured into a metal bat, soaked in an A5 size (148 × 210 mm) PET nonwoven fabric (05TH-80, manufactured by Hirose Paper Mfg Co., Ltd.) for 2 minutes, squeezed out the excess coating solution with a wringer, and then heated and dried at 80°C for 10 minutes to obtain a coated nonwoven fabric N1 in which particles were uniformly dispersed and coated. The coating mass was 3.23 $g/m^2$, and from the ICP analysis of the Pt amount on the coated product, the coating mass of the deodorizing catalyst B was 1.5 $g/m^2$, 1.8% by weight.

Deodorizing performance evaluation (trimethylamine)

A 20 $cm^2$ portion of the coated nonwoven fabric N1 corresponding to 3 mg of the deodorizing catalyst B was cut out. The deodorizing performance of trimethylamine was evaluated in the same manner as in the deodorizing performance evaluation of Example 16, except that the 20 $cm^2$ portion of the coated nonwoven fabric N1 was used instead of the deodorizing catalyst B. The relative concentration was 17%.

[Example 18]

Production of binder 2

Into a reaction container equipped with an stirrer, a reflux condenser, a dropping device, and a thermometer, 1000 g of ion-exchanged water and 0.5 g of disodium 4-dodecyl-2,4'-oxydibenzenesulfonate were charged and the temperature was raised to 75°C while nitrogen was replaced under stirring. The internal temperature was maintained at 75°C, 4 g of ammonium persulfate was added as a polymerization initiator, and after dissolution, an emulsion previously prepared by adding 7 g of acryl amide, 690 g of styrene, 6 g of divinylbenzene, and 15 g acrylic acid to 300 g of ion-exchanged water and 3.5 g of disodium 4-dodecyl-2,4'-oxydibenzenesulfonateunder stirring was continuously added dropwise to the reactant for 4 hours. After completion of dropping, aging was performed for 2 hours. The obtained aqueous solution containing the binder 2 was cooled to room temperature, and then adjusted to a solids content of 35% by weight and pH 8.0 by adding ion-exchanged water and an aqueous ammonium solution to obtain a 35% aqueous solution of binder 2.

Coating solution preparation/application (production of coated film N2)

By mixing and stirring 3 g of the deodorizing catalyst B obtained in Example 2, 5.7 g of the 35% binder 2 aqueous solution, 6 g of a 5% THICKENER SN615 (manufactured by San Nopco Ltd.) aqueous solution, 2 g of a 10% DOWFAX 2A1 (manufactured by Dow, Inc.) aqueous solution and 30 g of water, a coating solution in which particles were uniformly dispersed was obtained. Using an applicator with a slit width of 40 $\mu$m, with an automatic film applicator (manufactured by TESTER SANGYO CO., LTD.), the aqueous coating solution was applied onto a PET film (manufactured by TOYOBO CO., LTD., thickness: 25 $\mu$m) and then dried at 120°C for 10 minutes to obtain a coated film N2 in which particles were uniformly dispersed. The coating mass was 7.5 g/m$^2$, and the coating mass of the deodorizing catalyst B was 3.6 g/m$^2$.

Deodorizing performance evaluation (trimethylamine)

An 8 cm$^2$ portion of the coated film N2 corresponding to 3 mg of the deodorizing catalyst B was cut out. The deodorizing performance of trimethylamine was evaluated in the same manner as in the deodorizing performance evaluation of Example 16, except that the 8 cm$^2$ portion of the coated film N2 was used instead of the deodorizing catalyst B. The relative concentration was 25%.

[Example 19]

Coating solution preparation/application (production of coated nonwoven fabric N3)

By mixing and stirring 9 g of the deodorizing catalyst B obtained in Example 2, 6 g of 8% polyvinyl alcohol (Mw, Mw/Mn) aqueous solution and 9 g of water, an aqueous solution for coating in which particles were uniformly dispersed was obtained. Using an applicator with a slit width of 40 $\mu$m, with an automatic film applicator (manufactured by TESTER SANGYO CO., LTD.), the aqueous coating solution was applied onto a PET nonwoven fabric (05TH-80, manufactured by Hirose Paper Mfg Co., Ltd.) and then dried at 80°C for 10 minutes to obtain a coated nonwoven fabric N3 in which particles were uniformly dispersed. The coating mass was 25.8 g/m$^2$, and the coating mass of the deodorizing catalyst B was 24.5 g/m$^2$, 23.6% by weight.

Deodorizing performance evaluation (trimethylamine)

A 1.2 cm$^2$ portion of the coated nonwoven fabric N3 corresponding to 3 mg of the deodorizing catalyst B was cut out. The deodorizing performance of trimethylamine was evaluated in the same manner as in the deodorizing performance evaluation of Example 16, except that the 1.2 cm$^2$ portion of the coated nonwoven fabric N3 was used instead of the deodorizing catalyst B. The relative concentration was 14%.

[Comparative Example 14]

Coating solution preparation/application (production of coated film N4)

By mixing and stirring, 5.7 g of the 35% binder 2 aqueous solution, 6 g of a 5% THICKENER SN615 (manufactured by San Nopco Ltd.) aqueous solution, 2 g of a 10% DOWFAX 2A1 (manufactured by Dow, Inc.) aqueous solution and 30 g of water, a coating solution was obtained. Using an applicator with a slit width of 40 μm, with an automatic film applicator (manufactured by TESTER SANGYO CO., LTD.), the aqueous coating solution was applied onto a PET film (manufactured by TOYOBO CO., LTD., thickness: 25 μm) and then dried at 80°C for 10 minutes to obtain a coated film N4. The coating mass was 3.4 g/m$^2$.

Deodorizing performance evaluation (trimethylamine)

An 8 cm$^2$ portion of the coated film N4 was cut out. The deodorizing performance of trimethylamine was evaluated in the same manner as in the deodorizing performance evaluation of Example 16, except that the 8 cm$^2$ portion of the coated film was used instead of the deodorizing catalyst B. The relative concentration was 97%.

[Example 20]

Antibacterial evaluation

In accordance with JIS L1902, antibacterial evaluation was performed using Staphylococcus aureus (NBRC12732) and Escherichia coli (NBRC3301). A 1/20 nutrient broth medium was used as the test bacterial solution with a bacterial concentration of $1.5\times10^5$ (CFU/mL). A standard cotton cloth was used as a control.

In a vial, 0.4 g of the coated nonwoven fabric N3 obtained in Example 19 was placed, 0.2 ml of the test organism solution was dropped thereto, and the vial was capped and cultured at 37°C for 18 hours. The test bacteria were washed out from the test piece, and the number of viable bacteria in the washout solution was counted by counting the number of colonies of Escherichia coli by using ordinary agar medium (mixed plate culture method). The same operation was repeated three times, and the average values are shown in Tables 8 and 9. The antibacterial activity was calculated by the following formula. In addition, when the antibacterial activity is ≥ 2.0, it is determined that antibacterial activity is present.

```
Antibacterial activity = [Log (control sample, viable count

after culture) - Log (control sample, viable count

immediately after inoculation)] - [Log (test sample, viable

count after culture) - Log (test sample, viable count

immediately after inoculation)]
```

[Comparative Example 15]

The antibacterial properties were evaluated in the same manner as in Example 20, except that a nonwoven fabric (05TH-80, manufactured by Hirose Paper Mfg Co., Ltd.) was used instead of the coated nonwoven fabric N3. The results are shown in Tables 8 and 9.

[Example 21]

The coating solution obtained in Example 17 was spray-coated onto a PET nonwoven fabric (05TH-80, manufactured by Hirose Paper Mfg Co., Ltd.) using a small spray WIDER 1-10E1G (manufactured by ANEST IWATA Corporation) to obtain a coated nonwoven fabric N4 in which particles were uniformly dispersed and coated. The coating mass was 6.4 g/m$^2$, and the coating mass of the deodorizing catalyst B was 3.9 g/m$^2$, 4.6% by weight.

The antibacterial properties were evaluated in the same manner as in Example 20, except that the coated nonwoven fabric N4 was used instead of the coated nonwoven fabric N3. The results are shown in Table 8 and Table 9.

[Example 22]

The antibacterial properties were evaluated in the same manner as in Example 20, except that the coated nonwoven fabric N1 obtained in Example 17 was used instead of the coated nonwoven fabric N3. The results are shown in Table 8 and Table 9.

[Table 8]

Table 8 Antibacterial evaluation (Staphylococcus aureus)

| | Log (control sample, viable count) | | Log (test sample, viable count) | | Antibacterial activity value (B-A) -(D-C) |
|---|---|---|---|---|---|
| | immediately after inoculation A | after culture B | immediately after inoculation C | after culture D | |
| Example 20 | 4.44 | 7.43 | 4.44 | 1.30 | 6.1 |
| Example 21 | 4.52 | 7.10 | 4.52 | 1.30 | 5.8 |
| Example 22 | 4.52 | 7.10 | 4.52 | 1.30 | 5.8 |
| Comparative Example 15 | 4.44 | 7.43 | 4.48 | 4.21 | 3.3 |

[Table 9]

Table 9 Antibacterial evaluation (Escherichia coli)

| | Log (control sample, viable count) | | Log (test sample, viable count) | | Antibacterial activity value (B-A) -(D-C) |
|---|---|---|---|---|---|
| | immediately after inoculation A | after culture B | immediately after inoculation C | after culture D | |
| Example 20 | 4.48 | 7.49 | 4.48 | 1.3 | 6.2 |
| Example 21 | 4.50 | 7.47 | 4.55 | 1.30 | 6.2 |
| Example 22 | 4.50 | 7.47 | 4.50 | 1.30 | 6.2 |
| Comparative Example 15 | 4.48 | 7.49 | 4.50 | 5.82 | 1.7 |

[Example 23]

A 20 cm$^2$ portion of the coated nonwoven fabric N1 obtained in Example 17 was placed in a 3 L Tedlar bag (odor-removed product, manufactured by GL Sciences Inc) as a deodorizing product. 2.5 L of air through an activated carbon filter was introduced, and the inlet was capped with silicone. Then, 5.5 mL of trimethylamine/air at 20000 vol.ppm was injected into the sampling bag using a gas-tight syringe to adjust the trimethylamine concentration in the bag to about 45 ppm. The odor intensity was sensory evaluated at 80°C for 1 hour, 5 hours, and 24 hours stand. The odor intensity and evaluation method are as described in Example 11. The evaluation results are shown in Table 10.

[Comparative Example 16]

The same operation as in Example 23 was performed except that no deodorizing product was used, and the sensory evaluation results are shown in Table 10.

[Table 10]

Table 10

| | Deodorizing product | Sensory evaluation results | | |
|---|---|---|---|---|
| | | 1 h | 5 h | 24 h |
| Example 23 | coated nonwoven fabric N1 20 cm$^2$ | 1.5 | 0.5 | 0 |
| Comparative Example 16 | none | 4.5 | 4.5 | 5.0 |

INDUSTRIAL APPLICABILITY

The deodorizing catalyst, deodorant composition, and deodorizing product of the present invention can be suitably used for reduction and removal of an odor caused by odorants in the air, and can be particularly suitably used for, for example, deodorization in a room such as an automobile. The deodorizing catalyst, deodorant composition, and deodorizing product of the present invention can also be suitably used in uses in which antibacterial properties and antiviral properties are required. The deodorizing catalyst, deodorant composition, and deodorizing product of the present invention may be used as they are, or may be used in combination with other materials such as a resin in uses such as a resin-coated material, an interior sheet material, and an automobile inner panel. Further, the deodorizing catalyst, the deodorant composition, and the deodorizing product of the present invention can be applied to various uses without particular limitation, and can be applied to, for example, an upholstery material, a curtain, a carpet, a tile, a wallpaper, or an interior material for a vehicle.

**Claims**

1. A deodorizing catalyst comprising a composite oxide, and a metal component containing Pt supported on the composite oxide, wherein the catalyst has a degree of reduction represented by the following formula (1) in a range of 60% to 86%:

$$\text{degree of reduction (\%) = 100 - [X/Y] × 100}\qquad(1)$$

(in the formula (1), X represents a peak area obtained from a thermal conductivity detector (TCD) when the catalyst is dried and reduced by heating from 40°C to 400°C, and Y represents a peak area obtained from the TCD when an oxidation catalyst obtained by heat-treating the catalyst in an air or under oxygen atmosphere at 150°C for 2 hours and then cooling the catalyst to 40°C or lower is reduced by heating from 40°C to 400°C).

2. The deodorizing catalyst according to claim 1, wherein the metal component comprises Pt and at least one metal element selected from Re, Fe, Ru, Co, Rh, Ir, Ni, Pd, Cu, Ag, and Au.

3. The deodorizing catalyst according to claim 1 or 2, wherein the metal component comprises an alloy containing Pt and at least one metal element selected from Re, Fe, Ru, Co, Rh, Ir, Ni, Pd, Cu, Ag, and Au.

4. The deodorizing catalyst according to claim 3, wherein the alloy forms a solid solution.

5. The deodorizing catalyst according to any one of claims 1 to 4, wherein the composite oxide is MFI type zeolite.

6. The deodorizing catalyst according to claim 5, wherein the MFI type zeolite has a silica/alumina ratio ($SiO_2/Al_2O_3$ (mol/mol)) of 40 or less.

7. A deodorant composition comprising the deodorizing catalyst according to any one of claims 1 to 6 and a chemical adsorbent.

8. A deodorizing product comprising the deodorizing catalyst according to any one of claims 1 to 6 or the deodorant composition according to claim 7.

9. The deodorizing product according to claim 8, wherein the deodorizing product is a deodorizing fiber, a deodorizing coating material, or a deodorizing sheet.

10. An upholstery material, a curtain, a carpet, a tile, a wallpaper, or an interior material for a vehicle, using the deodorizing product according to claim 8 or 9.

11. An antibacterial/antiviral deodorizing product comprising the deodorizing catalyst according to any one of claims 1 to 6 or the deodorant composition according to claim 7.

12. An upholstery material, a curtain, a carpet, a tile, a wallpaper, or an interior material for a vehicle, using the antibacterial/antiviral deodorizing product according to claim 11.

[Fig. 1]

### CHANGE IN PROPIONALDEHYDE CONCENTRATION OF EXAMPLE 4

[Fig. 2]

### CHANGE IN CARBON DIOXIDE CONCENTRATION DURING PROPIONALDEHYDE CONTINUOUS DECOMPOSITION TEST OF EXAMPLE 4

[Fig. 3]

CHANGE IN ETHYL ACETATE CONCENTRATION OF EXAMPLE 5

[Fig. 4]

CHANGE IN CARBON DIOXIDE CONCENTRATION DURING ETHYL ACETATE CONTINUOUS DECOMPOSITION TEST OF EXAMPLE 5

[Fig. 5]

CHANGE IN TRIMETHYLAMINE CONCENTRATION OF EXAMPLE 6

[Fig. 6]

CHANGE IN CO2 CONCENTRATION DURING TRIMETHYLAMINE
CONTINUOUS DECOMPOSITION TEST OF EXAMPLE 6

[Fig. 7]

CHANGE IN METHYL MERCAPTAN CONCENTRATION OF EXAMPLE 7

[Fig. 8]

CHANGE IN CO₂ CONCENTRATION DURING METHYL MERCAPTAN
CONTINUOUS DECOMPOSITION TEST OF EXAMPLE 7

[Fig. 9]

CHANGE IN TOLUENE CONCENTRATION OF EXAMPLE 8

[Fig. 10]

CHANGE IN CO₂ CONCENTRATION DURING TOLUENE CONTINUOUS
DECOMPOSITION TEST OF EXAMPLE 8

[Fig. 11]

CHANGES OVER TIME IN EACH ODORANT CONCENTRATION OF EXAMPLE 9

[Fig. 12]

**AVERAGE DECOMPOSITION RATE OF EACH ODORANT AFTER 30 HOURS**

[Fig. 13]

**RELATIONSHIP BETWEEN ODORANT CONCENTRATION AND ODOR INTENSITY**

**EP 4 223 413 A1**

<table>
<tr><td colspan="2">**INTERNATIONAL SEARCH REPORT**</td><td>International application No.<br>**PCT/JP2021/035817**</td></tr>
</table>

**A. CLASSIFICATION OF SUBJECT MATTER**

*B01J 29/44*(2006.01)i; *A61L 9/00*(2006.01)i; *A61L 9/01*(2006.01)i
FI:   B01J29/44 A; A61L9/00 C; A61L9/01 B

According to International Patent Classification (IPC) or to both national classification and IPC

**B. FIELDS SEARCHED**

Minimum documentation searched (classification system followed by classification symbols)

B01J21/00-38/74; A61L9/00-9/22;B01J20/00-20/28,20/30-20/34

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JST7580/ JSTChina (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | WO 2017/010472 A1 (TAIYO KAGAKU CO., LTD.) 19 January 2017 (2017-01-19)<br>claims, paragraphs [0016]-[0023], [0028]-[0029], [0033]-[0034], [0048]-[0054], examples 1, 2, table 2 | 1-12 |
| Y | JP 2005-47775 A (MATSUSHITA ELECTRIC IND. CO., LTD.) 24 February 2005 (2005-02-24)<br>claims 1-3, 8-10, paragraphs [0001], [0029]-[0032], experimental examples 1, 2 | 1-12 |
| Y | JP 2002-200150 A (CALSONIC KANSEI CORP.) 16 July 2002 (2002-07-16)<br>claims 1, 2, 5, 6, paragraph [0001] | 2-4, 7 |
| Y | WO 2019/027057 A1 (NAT. UNIV. CORP. HOKKAIDO UNIV.) 07 February 2019 (2019-02-07)<br>claim 1, paragraphs [0004], [0010] | 2-4 |
| Y | JP 52-147590 A (OSAKA GAS CO., LTD.) 08 December 1977 (1977-12-08)<br>claims, page 1, lower left column, line 13 to lower right column, line 4, page 2, lower right column, lines 3-4, examples 1, 2 | 1-12 |

☐ Further documents are listed in the continuation of Box C.   ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **12 November 2021** | **30 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/035817**

| Patent document cited in search report | | | Publication date (day/month/year) | Patent family member(s) | Publication date (day/month/year) |
|---|---|---|---|---|---|
| WO | 2017/010472 | A1 | 19 January 2017 | (Family: none) | |
| JP | 2005-47775 | A | 24 February 2005 | (Family: none) | |
| JP | 2002-200150 | A | 16 July 2002 | (Family: none) | |
| WO | 2019/027057 | A1 | 07 February 2019 | (Family: none) | |
| JP | 52-147590 | A | 08 December 1977 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019027057 A
- JP 2015213908 A
- JP 2002200150 A
- JP 2000312710 A